(19) Europäisches Patentamt
European Patent Office
Office européen des brevets

(11) **EP 4 671 853 A1**

(12) **EUROPEAN PATENT APPLICATION**

(43) Date of publication:
**31.12.2025 Bulletin 2026/01**

(21) Application number: **24183971.1**

(22) Date of filing: **24.06.2024**

(51) International Patent Classification (IPC):
***G02C 7/04*** *(2006.01)*     ***G02C 11/00*** *(2006.01)*
***A61B 3/113*** *(2006.01)*

(52) Cooperative Patent Classification (CPC):
**G02C 7/04; A61B 3/113; G02C 11/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Imec VZW**
**3001 Leuven (BE)**

• **Universiteit Gent**
**9000 Gent (BE)**

(72) Inventor: **DE SMET, Herbert**
**9070 Destelbergen (BE)**

(74) Representative: **AWA Sweden AB**
**Box 5117**
**200 71 Malmö (SE)**

(54) **A CONTACT LENS, A CONTACT LENS SYSTEM, AND A METHOD FOR DETERMINING A POSITION OF THE CONTACT LENS**

(57)     According to an aspect there is provided a contact lens system comprising:

at least one magnet to be arranged at an eyelid of a subject for moving with the eyelid;

a contact lens to be arranged on an eyeball, the contact lens comprising a loop antenna, wherein the loop antenna is to be arranged for intersecting with a magnet trajectory of the magnet, during a blink movement of the eyelid, wherein the magnet passes over at least one edge of the loop antenna, causing a change of magnetic flux through the loop antenna;

a sensor for sensing at least one pulse during the blink movement in response to the change of magnetic flux through the loop antenna; and

a processing unit configured to receive data from the sensor representing the pulse, and to analyze the data for determining a position of the contact lens with respect to the magnet trajectory of the magnet.

Fig. 1

EP 4 671 853 A1

## Description

<u>Technical field</u>

The present disclosure relates to smart contact lenses, and more specifically to a contact lens, a contact lens system and a method for determining a position of the contact lens.

<u>Background</u>

Conventional contact lenses may compensate for visual impairment by providing a fixed dioptric power to an eye of an individual. However, for individuals suffering from for example presbyopia a fixed dioptric power may not be sufficient for compensating the visual impairment for various focal distances.

Smart contact lenses may offer ways of artificially mimicking various functions of an eye. For example, smart contact lenses may be able to offer a switch between two or more dioptric powers of the contact lens, depending on whether the individual is focusing in the near field or in the far field. This may be helpful to individuals which suffer from problems like presbyopia. However, determining the intended focal distance of an individual poses challenges.

One way of determining whether the eye is trying to focus in the near field may be to detect the tension in the ciliary muscles that try to change the shape of the crystalline lens in the eye. However, such measurements are complicated, and may not necessarily show high accuracy.

Another way of determining the intended focal distance of the eyes of an individual may be to use cameras attached to spectacles worn by the user. The cameras may measure the viewing angle of the eyes, and the information may be wirelessly sent to the contact lenses. However, the approach is not very practical to make a smart contact lens work. For example, a common motivation for using contact lenses is to avoid having to use spectacles. Further, the use of cameras makes the approach both bulky and expensive.

Hence, there is a need in the art for further improvements related to smart contact lenses.

<u>Summary</u>

An objective of the present disclosure is to mitigate, alleviate or eliminate one or more of the above-identified deficiencies in the art and disadvantages singly or in any combination. These and other objectives are at least partly met by the invention as defined in the independent claims. Preferred embodiments are set out in the dependent claims.

According to a first aspect there is provided a contact lens system comprising:

at least one magnet configured to be arranged at an eyelid of a subject for moving with the eyelid;

a contact lens configured to be arranged on an eyeball, the contact lens comprising a loop antenna, wherein the loop antenna is configured to be arranged for intersecting with a magnet trajectory of the at least one magnet, during a blink movement of the eyelid, wherein the at least one magnet passes over at least one edge of the loop antenna, causing a change of magnetic flux through the loop antenna;

a sensor configured for sensing at least one pulse during the blink movement in response to the change of magnetic flux through the loop antenna; and

a processing unit configured to receive data from the sensor representing the at least one pulse, and to analyze the data for determining a position of the contact lens with respect to the magnet trajectory of the at least one magnet.

By way of example, the eyelid may typically be an upper eyelid of the subject, however it may alternatively be a lower eyelid of the subject. The term "blink movement" as used herein thus refers to a movement of the eyelid for closing the eye and opening the eye again, thus a closing movement and an opening movement of the eyelid.

For simplicity the present disclosure generally discusses only one eyelid of one eye. However, it serves to mention that the subject typically has two eyes with respective eyelids. Thus, it is conceivable that at least one magnet may be arranged on two eyelids associated with each of the two eyes, and that a contact lens may be arranged on each of the two eyeballs.

The at least one magnet may be provided in a size small enough to be able to be arranged at, as for example on, an eyelid, without causing discomfort to the subject. Each magnet of the at least one magnet may be provided with a cross-sectional shape being square, rectangular, circular, elliptical or any other suitable shape.

According to an embodiment, the at least one magnet is a neodymium magnet. Neodymium magnets have the advantage of providing a strong magnetic flux, and are therefore capable of inducing pulses in the loop antenna even while keeping the magnet size small. However, it serves to mention that other strong magnets may alternatively be used. By way of example, the at least one magnet may be a Samarium-Cobalt magnet.

The contact lens is configured to be worn by a subject in such a way that the contact lens is arranged on the eyeball of the subject, mainly on the cornea of the eyeball. Thus, the at least one magnet, being arranged for example on an outer side of the eyelid, may be arranged very close to the contact lens and thus to the loop antenna. During a blink movement of the eyelid, the at least one magnet follows the magnet trajectory and may pass over, and very close to at least one edge of the loop antenna of the contact lens. By the present arrangement, the magnetic flux of the at least one magnet may affect the loop antenna, to generate for example an electric current or voltage by magnetic induction.

The loop antenna may be a loop of an electrically conducting material, arranged in or on the contact lens. By way of example, the loop antenna may extend along or close to a perimeter of the contact lens. In this manner, a large area, compared to the size of the contact lens, may be encompassed by the loop antenna. However, it is conceivable that the loop antenna may be arranged differently, for example close to a specific edge of the contact lens.

The loop antenna may comprise a single turn of the loop. However, typically, the loop antenna comprises a plurality of turns of the loop. By way of example, the loop antenna may comprise 5, 10, 14, 15, 20, or 25 turns. The loop antenna may further comprise two end terminals, one at each end of the loop antenna.

The at least one pulse may be induced by magnetic induction into the loop antenna. By way of example, as the magnetic flux through the loop changes, a change in electric current, electric voltage, or a combination thereof may be induced in the loop antenna. The at least one pulse may be referred to as an electromotive force pulse. Whether the induced pulse is a voltage pulse, a current pulse or a combination thereof may depend on the electrical load that is connected to the end terminals of the loop antenna. By way of example, if the sensor connected to the loop antenna is a high impedance voltage sensor, a voltage waveform may be induced. By way of further example, if the sensor connected to the loop antenna is instead a low impedance current sensor, a current waveform may be induced.

When a magnet passes over the at least one edge to enter the loop antenna a pulse may be induced. Similarly, when a magnet passes over the at least one edge to exit the loop antenna another pulse, with inverted polarity, may be induced. Further, the polarity of the pulses may also depend on the orientation of the magnet's poles with respect to the loop antenna. Put differently, if the north pole of the magnet is facing a plane of the loop antenna, the induced pulse may have inverted polarity as compared to if the south pole of the magnet is facing the plane of the loop antenna.

The at least one pulse during the blink movement may be induced in the loop antenna during the eyelid closing movement. By way of example, the magnetic flux through the loop antenna may thus change once through the loop antenna during the closing of the eye lid. Changing the magnetic flux through the loop antenna during the closing of the eyelid may be achieved by arranging a single magnet at the eyelid. The single magnet may, during the closing of the eyelid, pass over an edge, e.g. an upper edge, of the loop antenna, causing a change of magnetic flux through the loop antenna, in response to which the at least one pulse is induced.

It should be realized that during the opening movement of the eyelid, a corresponding pulse may be induced, which may have inverted polarity as compared to the pulse induced during the closing movement of the eyelid. The reason for the inversion of the pulse is that the change of magnetic flux is inverted. For example, when a magnet passes over an edge of the loop antenna from outside to inside the loop antenna, during a closing movement of the eyelid, the magnetic flux through the loop antenna may be increased. Thus, when a magnet passes over the same edge of the loop antenna, during an opening movement of the eyelid, the magnet moves in the opposite direction and instead moves from being inside to outside the loop antenna. Hence, the magnetic flux through the loop antenna may be decreased.

The contact lens may optionally comprise a light sensor. The light sensor may sense the amount of light reaching the contact lens. From the signal measured by the light sensor it may be determined whether the eyelid is closed or open. Thus, it may be determined whether the at least one pulse is induced during a closing or an opening of the eyelid. By the present arrangement, the processing unit may be configured to ignore the at least one pulse induced during an eyelid opening movement, and thus analyzing the data from an eyelid closing movement for determining the position of the contact lens with respect to the magnet trajectory of the at least one magnet.

The processing unit may be integrated in the contact lens. The processing unit may receive data from a sensor (which may also be integrated in the contact lens), and may analyze the data for determining the position of the contact lens with respect to the at least one magnet.

In this regard, the processing unit may for instance comprise a general-purpose processing unit, which may be provided with instructions for performing the analysis. Alternatively, the processing unit may be implemented as firmware arranged e.g. in an embedded system, or as a specifically designed processing unit, such as an Application-Specific Integrated Circuit (ASIC) or a Field-Programmable Gate Array (FPGA), which may be configured to implement functionality of the processing unit.

However, it should be realized that the processing unit may alternatively be external to the contact lens. The contact lens may include a wireless communication unit, which communicates with an external processing unit for transmitting the data to the processing unit. For instance, the processing unit may be arranged in a device which may be worn by the subject. Having the processing unit external to the contact lens may decrease complexity of the contact lens and manufacturing costs. It is foreseen that the contact lens needs to be replaced at regular intervals, whereas a processing unit arranged in an external unit may have a much longer lifetime. Also, a processing unit in an external unit may simplify receiving input or updates to the processing unit.

According to an embodiment, the position of the contact lens with respect to the at least one magnet is related to a viewing angle of the eye.

The at least one magnet is arranged at the eyelid and may move with the eyelid. During blink movements, the at least one magnet may move along the same magnet trajectory. The movement of the eyelid is typically a downward movement followed by an upward movement. Thus, during blink movements there is no substantial sideways movement of the eyelid and the at least one magnet.

The contact lens, on the other hand, may follow the movements of the eyeball. When the subject is watching something far away, i.e. focusing in the far field, the eyes may look straight ahead, and the contact lens may be in the middle of the eye. By way of example, if the loop antenna is provided as a circular antenna extending along or adjacent a perimeter of the contact lens, the magnet trajectory may intersect the loop antenna at the uppermost edge and the lowermost edge of the loop antenna. At this position the distance between the two edges at which the magnet trajectory intersects the loop antenna may be the largest, as for example the full diameter of the loop antenna. Consequently, the time difference between the two pulses induced when the at least one magnet passes the two edges of the loop antenna may be the largest.

When the subject is instead watching something at close range, i.e. focusing in the near field, the eyes may be angled inwards towards the nose, and the contact lens following the movement of the eyeball may be shifted to the side. Thus, the loop antenna is displaced sideways with respect to the magnet trajectory.

The magnet trajectory may intersect the loop antenna at a first edge of the loop antenna and a second edge of the loop antenna, located to a side of the loop antenna. At this position the magnet trajectory may intersect the loop antenna where the distance between the first edge and the second edge is not the largest distance between edges of the loop antenna, and thus the distance is smaller than when the subject is looking straight ahead, focusing in the far field. Consequently, the time difference between the two pulses induced when the at least one magnet passes the two edges of the loop antenna may be smaller. Thus, by the present arrangement, the position of the contact lens, and thus the viewing angle of the eye may be determined, from which the focal distance of the subject, may be determined.

It serves to mention that the position of the contact lens with respect to the magnet trajectory, and thus the viewing angle and focal length of the eye, may be determined in other manners than based on a time difference. Given as non-limiting example, the position of the contact lens may be determined based on a shape of the at least one pulse, as for example a rise time of the at least one pulse.

An advantage is that an easy manner may be provided of determining the position of the contact lens with respect to the magnet trajectory of the at least one magnet, and thus also the viewing angle and focal distance of the eye, without the need for complicated measurements of tension in the ciliary muscles, or bulky and expensive cameras attached to spectacles worn by the subject. Further, a more comfortable manner of determining the position of the contact lens may be provided, since the weight of the contact lens as well as the weight of the magnet may be kept low.

Another advantage with a contact lens comprising a loop antenna having a plurality of turns of the loop is that a loop antenna being more sensitive to changes in magnetic flux may be provided. Each turn of the plurality of turns of the loop may contribute to the induced pulse. Therefore, more turns of the loop may result in a stronger induced pulse, which may be more easily detected. Further, since the loop antenna is more sensitive to changes in magnetic flux, a smaller magnet may be used, while still providing a good detection of position of the contact lens with respect to the magnet trajectory. This in turn may be even more comfortable to the subject.

According to an embodiment, the processing unit is further configured to send a control signal, based on the position of the contact lens with respect to the magnet trajectory of at least one magnet, to the contact lens.

It serves to mention that in case the processing unit is external to the contact lens, the control signal may be sent from the external processing unit to the contact lens. By way of example, the control signal may be wirelessly transmitted to the contact lens. On the other hand, in case the processing unit is integrated in the contact lens, the control signal may be sent from the processing unit of the contact lens to another part of the contact lens. By way of example, the control signal may be sent by wired or wireless transmission.

According to an embodiment, the contact lens is configured to receive the control signal, and in response to the control signal change a dioptric power of the contact lens.

In order to change the dioptric power, the contact lens may comprise an electro-optic element. The electro-optic element may be arranged in the contact lens such that, when the contact lens is worn by the subject, the electro-optic element is arranged at or around the center of the cornea, in front of the pupil of the eye. The electro-optic element may be transparent to several wavelengths in the visible range, such that the subject wearing the contact lens may see clearly through the electro-optic element of the contact lens.

The electro-optic element may be made of a material with strong electro-optic effect such that when an electric field is applied to the electro-optic element, an optical property of the material may change. By way of example, when applying an electric field to the electro-optic element, a thickness, a curvature, a refractive index, and/or an optical path length through the electro-optic element may be changed. By the present arrangement, a contact lens with variable dioptric power may be provided. By varying the applied electric field, the change in the optical property, and thus also the dioptric power of the contact lens, may be tunable.

An advantage with this embodiment is that a contact lens may be provided that may improve the vision of the subject, in that the dioptric power may be automatically adapted in dependence of focal distance. The contact lens may be suitable to be used by subjects suffering from presbyopia.

According to an embodiment, the loop antenna extends along a perimeter of the contact lens.

In this manner, a large area, compared to the size of the contact lens, may be encompassed by the loop antenna. The larger the area encompassed by the contact lens, the larger magnetic flux may be provided for the same electric field.

An advantage with this embodiment is that by having the loop antenna extending close to the perimeter of the contact lens, the loop antenna may be kept out of the way of the optical area of the eye, i.e. the cornea and/or the pupil area of the eye. By the present arrangement, a contact lens with a loop antenna may be provided that does not interfere with the vision of the wearer of the contact lens.

According to an embodiment, the processing unit is configured to determine the position of the contact lens based on a shape of the at least one pulse.

The shape of the at least one pulse may be depending on the shape of the at least one magnet, the shape of the loop antenna and the position of the contact lens with respect to the magnet trajectory. Thus, by analyzing the shape of the at least one pulse, wherein the shape of the at least one magnet and the shape of the loop antenna are known, the position of the contact lens may be determined.

Given as a non-limiting example, in case the loop antenna has a circular shape, and each magnet of the at least one magnet has a square shape, the position of the contact lens may be determined based on a rise time of the at least one pulse. Optionally, the position of the contact lens may be determined based on the time between two pulses divided by the rise time of one of the pulses. Given as another non-limiting example, in case each magnet of the at least one magnet has a circular shape, the position of the contact lens may be determined based on a full width at half maximum (FWHM) of one pulse. Optionally, the position of the contact lens may be determined based on a full width at half maximum (FWHM) of one pulse divided by the time between two pulses. Given as yet another non-limiting example, in case each magnet of the at least one magnet has a circular shape, the position of the contact lens may be determined based on a skewness of one pulse.

According to an embodiment, the processing unit is configured to determine the position of the contact lens based on a rise time of the at least one pulse.

According to an embodiment, the sensor is configured for sensing at least two pulses during the blink movement in response to the change of magnetic flux through the loop antenna; and
the processing unit is configured to receive data from the sensor representing the at least two pulses, and to analyze the data for determining a position of the contact lens with respect to the magnet trajectory of the at least one magnet.

The at least two pulses during the blink movement may be induced in the loop antenna during the eyelid closing movement. The magnetic flux through the loop antenna may thus change twice through the loop antenna during the closing of the eye lid.

By way of example, changing the magnetic flux through the loop antenna twice during the closing of the eyelid may be achieved by arranging at least two magnets, a first magnet and a second magnet, at the eyelid. The first and the second magnets may thus, during the closing of the eyelid, pass over an edge of the loop antenna one after the other, causing a change of magnetic flux through the loop antenna, in response to which the at least two pulses are induced.

By way of further example, changing the magnetic flux through the loop antenna twice during the closing of the eyelid may be achieved by arranging a single magnet at the eyelid. The single magnet may, during the closing of the eyelid, pass over a first edge of the loop antenna and a second edge, e.g. an upper and a lower edge, of the loop antenna, one after the other, causing a change of magnetic flux through the loop antenna, in response to which the at least two pulses are induced.

It should be realized that during the opening movement of the eyelid, corresponding pulses may be induced, which may have inverted polarity as compared to the pulses induced during the closing movement of the eyelid. The reason for the inversion of the pulses is that the change of magnetic flux is inverted. For example, when a magnet passes over an edge of the loop antenna from outside to inside the loop antenna, during a closing movement of the eyelid, the magnetic flux through the loop antenna may be increased. Thus, when a magnet passes over the same edge of the loop antenna, during an opening movement of the eyelid, the magnet instead moves from being inside to outside the loop antenna. Hence, the magnetic flux through the loop antenna may be decreased.

According to an embodiment, the processing unit is configured to determine the position of the contact lens based on a time difference between the at least two pulses.

According to an embodiment, the at least one magnet comprises a first magnet and a second magnet, and the first magnet and the second magnet are configured to be arranged at the eyelid such that the first magnet and the second magnet, during the blink movement of the eyelid, follow the same magnet trajectory and such that the first magnet and the second magnet pass over the at least one edge of the loop antenna one after the other, causing a change of magnetic flux through the loop antenna, in response to which the at least two pulses are induced.

The second magnet may be arranged above the first magnet at the eyelid such that the first and second magnets are arranged along a substantially vertical line. By the present arrangement, the first magnet and the second magnet may follow the same magnet trajectory and thus may pass over the at least one edge of the loop antenna at the same location.

By way of example, the first magnet and the second magnet may be two separate magnets, each of which may be individually arranged at the eyelid.

By way of further example, the first and second magnets may alternatively be arranged into a common physical unit, such that the first and second magnets are jointly arranged at the eyelid. In this manner, the first and the second magnets may be provided with a desired distance between one another. By arranging the first magnet and the second magnet in a common physical unit, the first and second magnets may be configured to be arranged at the eyelid so that the common physical unit needs to be arranged with a correct orientation on the eyelid, without the need for arranging the first and second magnets individually in order to provide the correct magnet orientations as well as the correct distance between the magnets.

By way of example, if case square magnets are used, the position of the loop antenna with respect to the magnet trajectory may be determined by the time between the two pulses divided by the rise time of one pulse.

By way of example, in case circular magnets are used, the position of the loop antenna with respect to the magnet trajectory may be determined by the full width at half maximum (FWHM) of one pulse divided by the time difference between the two pulses.

By the present arrangement, the time between the two pulses, one pulse for each magnet, may represent a speed of the eyelid, and thus the magnets. The information on speed may be used for example to improve the accuracy determining the position of the contact lens based on the shape of a pulse, such as the rise time or the FWHM of a pulse. Thus, by the present arrangement, the accuracy of determining the position of the contact lens may be improved.

Moreover, the first magnet and the second magnet may have the same size and shape. However, it is conceivable that the two magnets may be configured differently. By way of example, the second magnet may be provided with a different height than the first magnet. For example, the second magnet may have half the height of the first magnet. The present arrangement may yield additional information that may be used to distinguish between eyelid closing (wide pulse followed by narrow pulse) and eyelid opening (narrow pulse followed by wide pulse).

An advantage with this embodiment is thus that a contact lens system with improved accuracy of determining the position of the contact lens may be provided.

According to an embodiment, the at least one magnet further comprises a third magnet configured to be arranged at the eyelid such that the third magnet, during the blink movement of the eyelid, follows a different magnet trajectory than the first magnet and the second magnet, and to pass over the at least one edge of the loop antenna at a different location from a location at which the first and second magnets pass over the at least one edge of the loop antenna, causing a change of magnetic flux through the loop antenna.

By way of example, the third magnet may be horizontally displaced at the eyelid with respect to the first and second magnets. By having the third magnet horizontally displaced with respect to the first and second magnets, the third magnet may follow a different magnet trajectory than that of the first and second magnets. The present arrangement may allow for distinguishing between whether the eye is shifted towards the nose or away from the nose.

An advantage with this embodiment is that, by being able to distinguish between whether the eye is shifted towards the nose or away from the nose, the processing unit may determine whether or not a change of dioptric power is needed. If it is determined that the eye is displaced inwards towards the nose, the subject may be focusing in the near field, and a change in dioptric power may be needed. However, if it is determined that the eye is instead displaced outwards, away from the nose, the subject is likely not focusing in the near field, and thus a change of dioptric power is likely not needed.

It is conceivable that the third magnet, horizontally displaced with respect to the first magnet, may be arranged at the eyelid, with only the first magnet, but not the second magnet, being arranged at the eyelid. Thus, a configuration with a first magnet and a third magnet, horizontally displaced with respect to the first magnet, may allow the processing unit to distinguish between whether the eye is shifted towards the nose or away from the nose.

According to an embodiment, the at least one magnet comprises a single magnet, and the single magnet is configured to be arranged at the eyelid such that the single magnet, during the blink movement of the eyelid, follows the magnet trajectory to pass over a first edge of the loop antenna and a second edge of the loop antenna, one after the other, causing a change of magnetic flux through the loop antenna, in response to which the at least two pulses are induced.

As previously mentioned, the time between two pulses may be related to the position of the lens with respect to the magnet trajectory in the following manner. When the subject is focusing in the far field, the magnet trajectory may intersect the loop antenna at the first and second edges, here being the uppermost edge and the lowermost edge of the loop antenna, where the distance between the two edges may be the largest. On the other hand, when the subject is instead focusing in the near field, the eyes may be angled inwards towards the nose, and the contact lens may thus be shifted to the side. The magnet trajectory may intersect the loop antenna at the first edge and the second edge, now located to a side of the loop antenna, where the distance between the two edges is typically smaller than when the subject is looking straight ahead, focusing in the far field. Consequently, the time difference between the two pulses induced when the single magnet passes the two edges of the loop antenna may be related to the position of the contact lens. Thus, from the time difference, the viewing angle of the eye and the focal distance of the subject may be determined.

According to a second aspect there is provided a contact lens configured to be arranged on an eyeball, the contact lens comprising:

a loop antenna, wherein the loop antenna is configured to be arranged for a magnet trajectory of at least one magnet moving with an eyelid during a blink movement, to pass over at least one edge of the loop antenna, causing a change of magnetic flux through the loop antenna; and
a sensor configured for sensing at least one pulse during the blink movement in response to the change of magnetic flux through the loop antenna.

The contact lens, and/or the sensor of the contact lens, may be configured to transmit data from the sensor representing the at least one pulse. By way of example, the data may be transmitted to a processing unit that may either be arranged in the contact lens or is external to the contact lens. Transmitting the data enables analyzing the data for determining the position of the contact lens with respect to the at least one magnet.

According to an embodiment, the contact lens further comprises:
a processing unit configured to receive data from the sensor representing the at least one pulse, and to analyze the data for determining a position of the contact lens with respect to the at least one magnet.

According to a third aspect there is provided a method for determining a position of a contact lens with respect to at least one magnet of a contact lens system, wherein the at least one magnet is arranged at an eyelid of a subject for moving with the eyelid, and wherein the contact lens is arranged on an eyeball, the contact lens comprising a loop antenna, the method comprising:

passing, during a blink movement of the eyelid, the at least one magnet along a magnet trajectory over at least one edge of the loop antenna, causing a change of magnetic flux through the loop antenna;
sensing, by a sensor, at least one pulse during the blink movement in response to the change of magnetic flux through the loop antenna;
receiving, by a processing unit, data from the sensor representing the at least one pulse; and
analyzing, by the processing unit, the data for determining a position of the contact lens with respect to the at least one magnet.

According to a fourth aspect there is provided a processing unit for determining a position of a contact lens with respect to at least one magnet of a contact lens system, the processing unit being configured to:

receive data, wherein the data represents at least one pulse induced in response to a change of magnetic flux through a loop antenna of the contact lens and sensed by a sensor, wherein the change of magnetic flux through the loop antenna is caused by the at least one magnet being passed along a magnet trajectory over at least one edge of the loop antenna, during a blink movement of the eyelid, while the at least one magnet is arranged at an eyelid of a subject for moving with the eyelid, and while the contact lens is arranged on an eyeball; and
analyze the data for determining a position of the contact lens with respect to the at least one magnet.

According to an embodiment, the processing unit is configured to determine the position of the contact lens based on a shape of the at least one pulse.

According to an embodiment, the processing unit is configured to determine the position of the contact lens based on a rise time of the at least one pulse.

According to an embodiment, the data represents at least two pulses induced in response to a change of magnetic flux through a loop antenna of the contact lens and sensed by a sensor, wherein the change of magnetic flux through the loop antenna is caused by the at least one magnet being passed along a magnet trajectory over at least one edge of the loop antenna, during a blink movement of the eyelid, while the at least one magnet is arranged at an eyelid of a subject for moving with the eyelid, and while the contact lens is arranged on an eyeball.

According to an embodiment, the processing unit is configured to determine the position of the contact lens based on a time difference between the at least two pulses.

According to a fifth aspect there is provided a computer program product comprising a computer-readable storage medium storing computer-readable instructions which, when executed by a processing unit, will cause the processing unit to perform a method comprising:

receiving data, wherein the data represents at least one pulse induced in response to a change of magnetic flux through a loop antenna of the contact lens and sensed by a sensor, wherein the change of magnetic flux through the loop antenna is caused by the at least one magnet being passed along a magnet trajectory over at least one edge of the loop antenna, during a blink movement of the eyelid, while the at least one magnet is arranged at an eyelid of a subject for moving with the eyelid, and while the contact lens is arranged on an eyeball;

analyzing the data for determining a position of the contact lens with respect to the at least one magnet.

Effects and features of the second, third, fourth and fifth aspects are largely analogous to those described above in connection with the first aspect. Embodiments mentioned in relation to the first aspect are largely compatible with the second, third, fourth and fifth aspects. It is further noted that the disclosure relates to all possible combinations of features unless explicitly stated otherwise.

Other objectives, features and advantages of the present disclosure will appear from the following detailed description, from the attached claims as well as from the drawings.

Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.

Fig. 1 schematically illustrates a contact lens system comprising a contact lens and a magnet.

Fig. 2A schematically illustrates the situation wherein the subject is watching something far away, i.e. focusing in the far field, with the eyes looking straight ahead.

Fig. 2B schematically illustrates the situation wherein the subject is focusing at an intermediate distance.

Fig. 2C schematically illustrates the situation wherein the subject is watching something at close range, i.e. focusing in the near field.

Fig. 3 schematically illustrates further details of the contact lens comprising an electro-optic element for enabling change of the dioptric power.

Fig. 4A schematically illustrates a contact lens system, comprising two square shaped magnets arranged on the outside of an eyelid of a subject such that the magnets follow the same magnet trajectory during a blink movement.

Fig. 4B schematically illustrates the geometry related to the viewing angle, focal distance and the interpupillary distance of the eyes of the subject.

Fig. 4C schematically illustrates how the two magnets pass over the edge of the loop antenna, step by step, for one of the eyes.

Fig. 4D schematically illustrates the expected voltage waveform of pulses in response to the two magnets passing over the edge of the loop antenna.

Fig. 4E illustrates equation (2) plotted as a linear plot and a logarithmic plot.

Fig. 5A schematically illustrates a contact lens system, comprising two circularly shaped magnets arranged on the outside of an eyelid of a subject.

Fig. 5B schematically illustrates the expected voltage waveform of pulses in response to the two circular magnets passing over the edge of the loop antenna.

Fig. 6 illustrates a schematic block diagram shortly summarizing the method for determining a position of a contact lens with respect to at least one magnet of a contact lens system.

Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

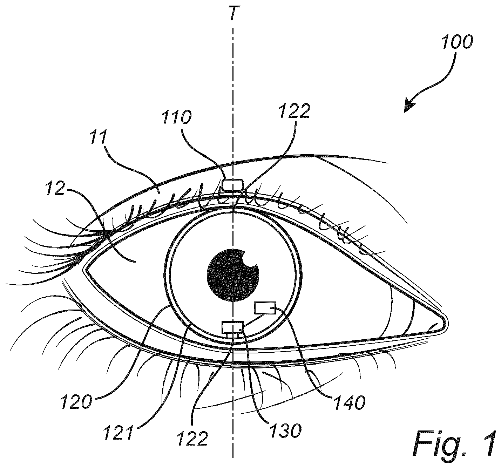

Fig. 1 schematically illustrates a contact lens system 100. The contact lens system 100 comprises a magnet 110 configured to be arranged on the outside of an eyelid 11 of a subject. The magnet 110 may be arranged centrally on the eyelid 11 and may move with the eyelid 11, as for example during a blink movement of the eyelid 11. By the present arrangement, the magnet 110 may follow a magnet trajectory during a blink movement, passing centrally over the cornea of the eyeball 12, when the subject looks straight ahead.

The contact lens system further comprises a contact lens 120. The contact lens 120 is configured to be arranged on the eyeball 12, thus worn on the cornea by the subject. The contact lens 120 comprises a loop antenna 121. In the present example, the loop antenna 121 is a circular loop antenna. The loop antenna 121 extends along a perimeter of the contact lens 120.

When the contact lens 120 is worn by the subject, the contact lens 120 is centered on the cornea of the eyeball 12. As previously described, when the subject looks straight ahead the magnet 110 may follow a magnet trajectory during a blink movement, passing centrally over the cornea of the eyeball 12. The magnet 110 may pass centrally over at least one edge 122, and in the present example two edges 122 of the loop antenna 121. Thus, the magnet trajectory may intersect with the loop antenna 121.

When the magnet 110 passes over an edge 122 of the loop antenna 121, the magnetic flux through the loop antenna 121 may be changed. The change of magnetic flux may cause a pulse to be induced by magnetic induction into the loop antenna 121, each time the magnet passes over an edge 122 of the loop antenna 121. By way of example, the pulse may be in the form of an electric current, an electric voltage, or a combination thereof.

The contact lens system 100 further comprises a sensor 130. In the present example, the sensor 130 is integrated into the contact lens 120. By way of example, the sensor 130 may be a current sensor or a voltage sensor. The sensor 130 may be connected to two end terminals of the loop antenna 121. By the present arrangement, the sensor 130 may sense the pulses during the blink movement in response to the change of magnetic flux through the loop antenna 121.

The contact lens system 100 further comprises a processing unit 140. The processing unit 140 is configured to receive data from the sensor 130 representing the pulses induced in the loop antenna 121. In the present example, the processing unit 140 is integrated into the contact lens 120. As such, the processing unit 140 may receive the data via a physical connection to the sensor 130. However, it is conceivable that the processing unit 140 may alternatively be external to the contact lens 121. In such case, the data may be wirelessly transmitted from the contact lens 120 to the processing unit 140.

The processing unit 140 is configured to analyze the data for determining a position of the contact lens 120 with respect to the magnet trajectory of the magnet 110. Determining the position of the contact lens 120 with respect to the magnet trajectory may be a manner of determining the viewing angle and focal distance of the subject. Determining the position of the contact lens 120 may be done in a number of different manners. Some examples will be discussed in the following.

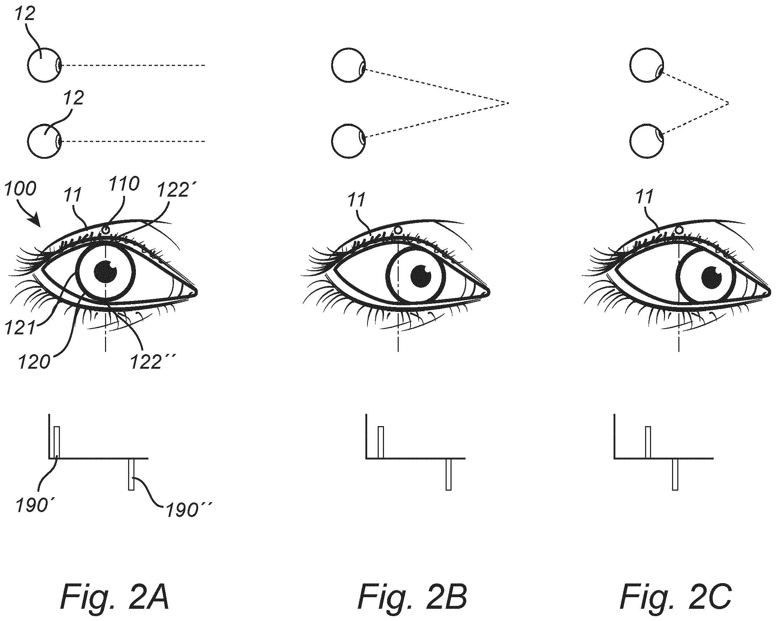

Figs 2A-2C schematically illustrate induction of pulses in the loop antenna 121 of the contact lens system 100, at three different viewing angles of the subject. The upper part illustrates a top view of the viewing angle of the eyeballs 12. The middle part illustrates the position of the eye and thus of the contact lens 120 with respect to the magnet trajectory. The lower part illustrates the induced pulses in the loop antenna 121 as a response to the change in magnetic flux through the loop antenna 121 a during blink movement, or more specifically during an eye closing movement of the eyelid 11.

Fig. 2A schematically illustrates the situation wherein the subject is watching something far away, i.e. focusing in the far field, with the eyes looking straight ahead. As illustrated, the contact lens 120 may be in the middle of the eye.

In the present example, the single magnet 110 is arranged centrally on the eyelid 11 such that the single magnet 110 follows the magnet trajectory to pass over a first edge 122' of the loop antenna 121 and a second edge 122" of the loop antenna 121, one after the other. Each time the magnet 110 passes an edge of the loop antenna 121, the magnetic flux through the loop antenna 121 changes, and a pulse is induced in the loop antenna 121 in response.

Thus, when the magnet 110 passes over the first edge 122' of the loop antenna 121 from being outside to inside the loop antenna 121, the magnetic flux through the loop antenna 121 may be increased. In response, a pulse 190' is induced. On the other hand, when the magnet passes over the second edge 122" of the loop antenna 121, such that the magnet 110 instead moves from being inside to outside the loop antenna 121, the magnetic flux through the loop antenna 121 may be decreased. In response a pulse 190" is induced. Since one passage causes an increase and the other passage causes a decrease of magnetic flux, the polarity of the pulses 190', 190" may be inverted. The time between the two pulses 190', 190" may depend on the speed of the magnet 110 during the blink movement. Although the speed of the magnet 110, or rather the speed of the eyelid 11, may vary from subject to subject, typically the speed is substantially the same for most blink movements of a specific subject. Thus, the time between the pulses 190', 190" can be expected to be substantially the same for most blink movements when the subject is focusing in the far field.

Fig. 2B schematically illustrates the situation wherein the subject is focusing at an intermediate distance. Typically, the magnet trajectory is unchanged when the subject changes focal distance. However, as illustrated here, the contact lens 120 may be slightly displaced from the middle of the eye, inwards towards the nose.

The displacement of the contact lens 120 has the effect that the magnet 110 following the magnet trajectory passes over the first edge 122' and the second edge 122" of the loop antenna 121 at different locations of the loop antenna 121, than when the subject is focusing in the far field. The distance between these locations may be shorter than when the subject is focusing in the far field. Thus, the time between the induced pulses may be shorter.

Fig. 2C schematically illustrates the situation wherein the subject is watching something at close range, i.e. focusing in the near field. As illustrated, the contact lens 120 may be clearly displaced from the middle of the eye, inwards towards the nose.

Due to the displacement of the contact lens 120, the magnet 110 following the magnet trajectory passes over the first edge 122' and the second edge 122" of the loop antenna 121 at different locations of the loop antenna 121, than when the subject is focusing in the far field or at an intermediate distance. The distance between these locations may be even shorter than when the subject is focusing at an intermediate distance. Thus, the time between the induced pulses may be shorter.

The sensor 130 may sense the two pulses 190', 190" during the blink movement in response to the change of magnetic flux through the loop antenna 121, and send the data to the processing unit 140.

The processing unit 140 receiving the data from the sensor 130 representing the two pulses 190', 190", may determine a position of the contact lens 120 with respect to the magnet trajectory of the at least one magnet 110. The processing unit 140 may be configured to determine the position of the contact lens 120 based on the time difference between the at least two pulses 190', 190". A longer time between the two pulses 190', 190" may be indicative of the subject focusing in the far field. A shorter time between the two pulses 190', 190" may be indicative of the subject focusing in the near field. By way of example, a threshold value for the time between the pulses 190', 190" may be determined, below which the subject may be considered to be focusing in the near field, and above which the subject may be considered to be focusing in the far field.

It serves to mention that a calibration for the individual subject may be required in order to adjust parameters such as blink speed and threshold value for the time difference between pulses.

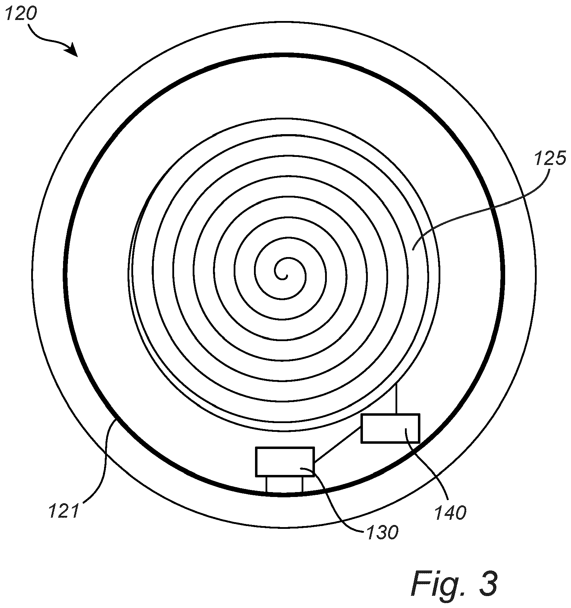

Fig. 3 schematically illustrates some further details of the contact lens 120.

Determining the intended focal distance of the subject, by determining the viewing angle from the position of the contact lens 120 with respect to the magnet trajectory of the at least one magnet 110, may be useful in several applications. One particular application is in case the subject suffers from presbyopia, or has other problems with the vision related to focusing. To that end the contact lens 120 may be configured to adapt its dioptric power, depending on the position of the contact lens 120.

The processing unit 140 may be configured to determine whether a change in dioptric power is needed, based on the position of the contact lens 120 with respect to the magnet trajectory. By way of example, in case the processing unit 140 determines that the lateral displacement of the contact lens 120 is small and that the subject is focusing in the far field, the processing unit 140 may determine that no change in dioptric power is needed. In other words, the inherent dioptric power of the contact lens 120 may be sufficient to provide a good focus for the subject when focusing in the far field. However, in case the processing unit 140 instead determines that the displacement of the contact lens 120 is large, for example above a threshold value, and that the subject is focusing in the near field, the processing unit 140 may determine that a change in dioptric power is needed. In other words, the inherent dioptric power of the contact lens 120 may be insufficient to provide a good focus for the subject when focusing in the near field.

The contact lens 120 may be configured to change the dioptric power. In order to change the dioptric power, the contact lens 120 may comprise electro-optic element 125. In the present example, the electro-optic element 125 is arranged at and/or around the center of the cornea, in front of the pupil of the eye. By applying an electric field to the electro-optic element 125 the dioptric power of the contact lens 120 may be varied. By way of example, a thickness, a curvature, a refractive index, and/or an optical path length through the electro-optic element 125 may be changed by applying an electric field.

The processing unit 140 may be configured to send a control signal to the electro-optic element 125. The control signal may be based on the determined position of the contact lens 120 with respect to the magnet trajectory. By the present arrangement, an electric field may be applied to the electro-optic element 125, and thereby the dioptric power of the contact lens 120 may be changed, based on the position of the contact lens 120.

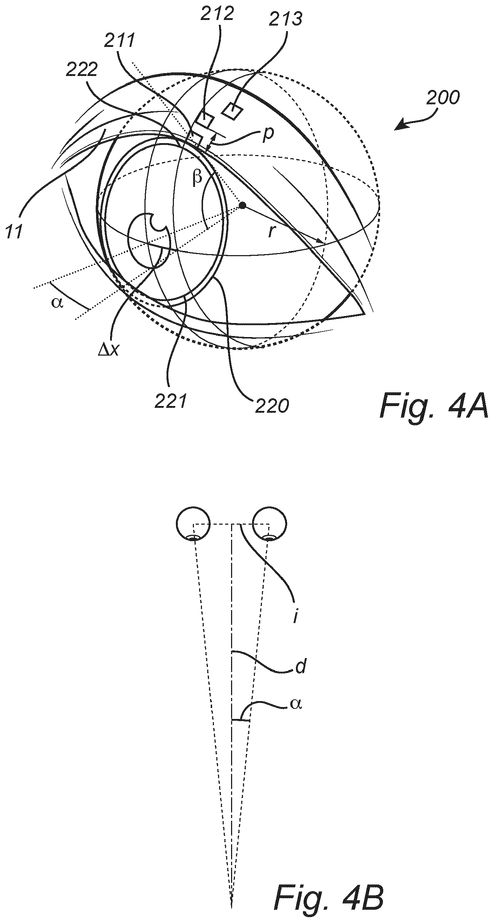

Figs 4A-4E schematically illustrate a contact lens system 200, comprising two magnets, i.e. a first magnet 211 and a second magnet 212, arranged on the outside of an eyelid 11 of a subject. The two magnets 211, 212 have a rectangular shape, and may more specifically have a square shape.

As illustrated in Fig. 4A, the first magnet 211 and the second magnet 212 may be arranged on the eyelid 11, one above the other, such that the two magnets 211, 212 follow the same magnet trajectory during a blink movement of the eyelid 11. By the present arrangement, the first magnet 211 and the second magnet 212 pass over the edge 222 of the loop antenna 221 one after the other. In the present example, the two magnets 211, 212 are arranged on the eyelid 11 so that they typically pass only one edge 222, in this case the upper edge 222 of the loop antenna 221. For each of the two magnets 211, 212, passing the edge 222 of the loop antenna 221 causes a change of magnetic flux through the loop antenna 221, in response to which two pulses are induced, on pulse by each of the magnets 211, 212.

The contact lens system 200 comprises a processing unit (not shown here), that may be integrated with the contact lens 220, or may alternatively be external to the contact lens 220. Since two pulses are induced, one by each of the two magnets 211, 212, the processing unit may be configured to determine the position of the contact lens 220 based on the time difference between the two pulses. In the present example, the processing unit may be further configured to determine the position of the contact lens 220 based on a shape of at least one pulse, and more specifically, based on a rise time of at least one pulse. A mathematical consideration thereof will now be presented.

In the following, an estimation is presented of the magnitude of the voltage level induced in the loop antenna 221 by a magnet 211, 212 attached to the eyelid 11 passing over the contact lens 220 with a multi-turn circular loop antenna 221.

The induced voltage may generally be expressed as:

$$U = n\frac{d\phi}{dt}$$

wherein $\phi$ is the magnetic flux entering the loop antenna 221 and $n$ is the number of turns of the loop antenna 221.

The speed at which the magnetic flux changes may be determined by the blink speed $v$, the width $w$ and the height $h$ of the magnet 211, 212, and the magnetic field $B$ generated by the magnet 211, 212:

$$\frac{d\phi}{dt} = \frac{whB}{h/v} = vwB$$

From these equations, the voltage may be expressed as:

$$U = nvwB$$

By way of example, the number of turns in the loop antenna 221 may be 14. By way of further example, a typical blink speed may be approximately 0.2 m/s.

From the equation of the voltage, it is realized that the height of the magnet is not relevant in the present approximate derivation. By way of example, the width of the magnet 211, 212 is assumed to be 2 mm. In order to estimate the magnetic field generated by the magnet 211, 212, a neodymium magnet is considered, which may be one of the strongest magnets having residual field strengths of up to 1.4 T. Usually, the external magnetic field may be smaller than this, since the magnetic field strength is reduced with increasing distance to the magnet 211, 212. The relevant parameter in the present example is the magnetic field strength at the posterior side of the eyelid 11. Here it is assumed that the eyelid 11 has an approximate thickness of around 0.5-1.0 mm. Therefore, a more realistic value of the magnetic field strength reaching into the loop antenna 221 is 0.2 T. The induced voltage may thus be estimated to be:

$$U = 14 \cdot 0.2 \cdot 0.002 \cdot 0.2 = 1.1\, mV$$

This voltage level may be easily and realistically measured by for example a sensor integrated in the contact lens 220.

Fig. 4B schematically illustrates the geometry related to the viewing angle, focal distance and the interpupillary distance of the eyes of the subject. In the following, an example approach is presented for deriving the intended focal distance from the measured voltage waveform comprising the two pulses.

Based on the illustrations in Fig. 4B as well as in Fig. 4A, the relationship between the intended focal distance d, the interpupillary distance $i$, the viewing angle $\alpha$, the eye radius $r$ and the eye displacement $\Delta x$ may be expressed as:

$$\tan \alpha = \frac{i}{2d} = \frac{\Delta x}{r} \quad \Leftrightarrow \quad \Delta x = \frac{ri}{2d} \quad \Leftrightarrow \quad d = \frac{ri}{2\Delta x}$$

A symmetric viewing is assumed, meaning that the viewing angle $\alpha$ for each of the eyes is half of the vergence angle between the eyes. From this equation it may be realized that the intended focal distance $d$ is inversely proportional to the eye displacement $\Delta x$. For a very short focal distance of 20 cm and for typical values for the eye radius $r$ = 11.5 mm and the interpupillary distance $i$ = 64 mm, it is found that $\Delta x$ = 1.8 mm. Thus, an eye displacement larger than 2.0 mm is unlikely to occur. Therefore, only values of the eye displacement lower than or equal to 2.0 mm will be considered.

Now returning to Fig. 4A, the relationship between the eye displacement $\Delta x$ and magnet trajectory of the magnets 211, 212 passing over the edge 222 of the loop antenna 221 will be considered.

In the present example, two small and identical magnets 211, 212 are arranged on the outside of the eyelid 11, above one another, so that the two magnets 211, 212 move along the same magnet trajectory. When the eyelid 11 closes, the first magnet 211 and the second magnet 212 may pass over the edge 222 to enter the loop antenna 221 area consecutively, leading to two voltage pulses. The time $\Delta t$ between the start of the two pulses depends on the eyelid closing speed $v$ and the vertical position difference $p$ of the two magnets 211, 212. The time between the start of the two pulses may be expressed as:

$$\Delta t = \frac{p}{v}$$

The shape of the voltage pulses depends on the size and shape of the magnets 211, 212, and on the eye displacement $\Delta x$, as will be described in the following paragraphs. It serves to mention that above a certain displacement $\Delta x$ the two pulses may overlap. However, as mentioned above, the maximum expected displacement $\Delta x$ is 2.0 mm. Thus, pulse overlap may be avoided by carefully selecting the size and position of the magnets, 211, 212 on the eyelid 11.

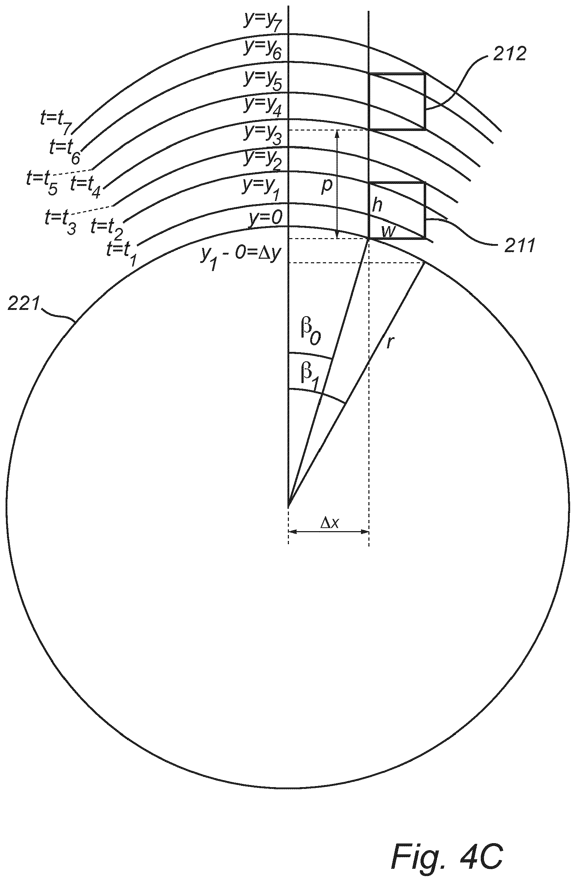

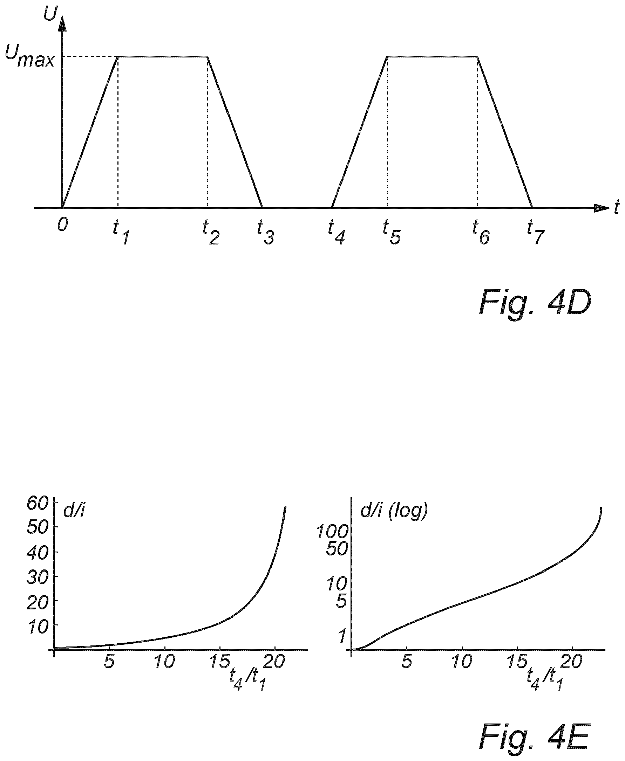

Fig. 4C schematically illustrates how the two magnets 211, 212 pass over the edge 222 of the loop antenna 221, step by step, for one of the eyes. The corresponding resulting pulses are illustrated in Fig. 4D. The shape of the voltage waveform with the pulses as a function of eye displacement $\Delta x$ will now be discussed.

Consider the case where the two magnets 211, 212 are identical in size and shape, thus the two magnets 211, 212 have a rectangular shape with a width w and a height $h$. The vertical distance between the bottom edge of each of the two magnets, 211, 212 is $p$, so that the net distance between the magnets 211, 212 is $p$ - $h$. The center of the eye pupil, and thus also the center of the loop antenna 221, is displaced a distance $\Delta x$ with respect to the magnet trajectory along which the magnets 211, 212 move. It may be assumed that the magnets 211, 212 move downwards with a constant speed $v$, i.e. the eyelid 11 closing speed. To maintain a clean and clear drawing, the magnets 211, 212 remain in the same location and instead different positions for the loop antenna 221 moving upwards are illustrated. Since the movement speed of the magnets 211, 212 is assumed to be constant ($v$), the corresponding upward shifts are over distances $y_i = v \cdot t_i$.

Eight moments in time are relevant for the discussion of the shape of the induces pulses, which are listed here:

- At $t = 0$, the bottom side of the first magnet 211 reaches the loop antenna 221. As illustrated in Fig. 4C, the left hand side of the bottom of the first magnet 211 may pass over the edge and enter the loop antenna 221 region first. It should be realized that for the other eye, the situation will be mirrored and so the right hand side may be first.
- At $t = t_1$, the other side, i.e. right hand side, of the bottom of the first magnet 211 has also reached the loop antenna 221.
- At $t = t_2$, the left hand side of the top of the first magnet 211 reaches the loop antenna 221.
- At $t = t_3$, the right hand side of the top of the first magnet 211 reaches the loop antenna 221. Thus, the first magnet 211 is now completely inside the circle defined by the loop antenna 221.
- At $t = t_4$, the left bottom side of the second magnet 212 reaches the loop antenna 221.
- At $t = t_5$, the right bottom side of the second magnet 212 reaches the loop antenna 221.
- At $t = t_6$, the left top side of the second magnet 212 reaches the loop antenna 221.
- At $t = t_7$, the right top side of the second magnet 212 reaches the loop antenna 221. Thus, both magnets 211, 212 are now inside the circle defined by the loop antenna 221.

First, it may be realized that $y_2 = h$, $y_4 = p$ and $y_6 = p + h$. To calculate $\Delta y = y_1$, consider the two indicated rectangular triangles with bottom corner in the center of the antenna at $t = 0$ and sharp angles $\beta_0$ and $\beta_1$, respectively. Thus, the following expression may be written:

$$r \cos \beta_0 = r \cos \beta_1 + \Delta y$$

$$r \sin \beta_0 = \Delta x$$

$$r \sin \beta_1 = \Delta x + w$$

By eliminating the angles and solving for $\Delta y$, it is found that:

$$t_1 v = y_1 = \Delta y = r \left( \cos \arcsin \frac{\Delta x}{r} - \cos \arcsin \frac{\Delta x + w}{r} \right) \qquad (1)$$

From the drawing it may be realized that $y_3 - y_2 = y_5 - y_4 = y_7 - y_6 = \Delta y$. Thus it may be concluded that:

$$(y_1, y_2, y_3, y_4, y_5, y_6, y_7) = (\Delta y, h, h + \Delta y, p, p + \Delta y, p + h, p + h + \Delta y)$$

wherein $\Delta y$ is given by equation (1).

Fig. 4D schematically illustrates the expected voltage waveform of pulses in response to the two magnets 211, 212 passing over the edge of the loop antenna 221. Each of the pulses show a certain rise time and a decay time, in between which a plateau is reached. However, it should be realized that this may be merely due to theoretical approximation. In a real contact lens system, the slanted lines might not be perfectly linear, because the loop antenna is curved. Between $t_1$ and $t_2$, and between $t_5$ and $t_6$, the speed with which the magnetic flux inside the loop antenna increases, is constant, leading to a constant voltage plateau $U_{max} = nBwv$.

The information about $\Delta x$, and hence the viewing angle and intended focal distance, is present in $t_1 = t_3 - t_2 = t_5 - t_4 = t_7 - t_6 = \Delta y/v$. This expression comprises the eyelid closing speed $v$, which may be determined from $t_4 = p/v$.

Now equation (1) may be solved for $\Delta x$. Dimensionless variables may be defined as

$$W = \frac{w}{r},\ T = \frac{t_1 v}{r} = \frac{p}{r} \cdot \frac{t_1}{t_4}\text{, and } X = \frac{\Delta x}{r}.$$ Equation (1) may now be written as:

$$T = (\cos \arcsin X - \cos \arcsin(X + W))$$

which can be solved for $X$:

$$X = \frac{\sqrt{4T^4 + 4T^2 W^2 - 2T^4 W^2 - T^2 W^4 - T^6} - W(T^2 + W^2)}{2(T^2 + W^2)}$$

By way of example, solving for $X$ may be done by using WolframAlpha, or any other suitable mathematical tool.

The intended focal distance $d$ may then be expressed as:

$$d = \frac{ri}{2\Delta x} = \frac{i}{2X} = \frac{(T^2 + W^2)}{\sqrt{4T^4 + 4T^2 W^2 - 2T^4 W^2 - T^2 W^4 - T^6} - W(T^2 + W^2)} \cdot i$$

which can be simplified to:

$$\frac{d}{i} = \left(\frac{2}{\sqrt{1 + \left(\frac{W}{T}\right)^2}} - W\right)^{-1}$$

or, by expanding $W$ and $T$:

$$\frac{d}{i} = \left(\frac{2}{\sqrt{1 + \left(\frac{w}{p} \cdot \frac{t_4}{t_1}\right)^2}} - \frac{w}{r}\right)^{-1} \tag{2}$$

Fig. 4E illustrates equation (2) plotted in a graph for the typical values $w = 2$ mm, $p = 4$ mm and $p = 11.5$ mm. Fig. 4E includes a linear plot and a logarithmic plot.

The graph has a vertical asymptote for $\frac{t_4}{t_1} = \frac{p}{w} \sqrt{4\left(\frac{r}{w}\right)^2 - 1}$, which corresponds to the situation $\Delta x = 0$, thus focal distance in infinity, i.e. far field.

The accuracy with which the intended focal distance may be determined depends on the accuracy with which the ratio $t_4/t_1$ may be determined. If simply switching between near field and far field focus is intended, it suffices to identify a desired transition distance (e.g. 40 cm) and determine the corresponding $t_4/t_1$ ratio. For an interpupillary distance of 6.4 cm and with w and $p$ chosen as above, this results in $t_4/t_1 = 11.8$.

Equation (2) may be inverted as:

$$\frac{t_4}{t_1} = \frac{p}{w} \sqrt{\left(\frac{2}{\frac{i}{d} + \frac{w}{r}}\right)^2 - 1}$$

By entering the desired transition point for $d$ into this equation, a corresponding ratio for $t_4/t_1$ may be found.

In a practical implementation, it may be advantageous to implement some hysteresis. By way of example, switching to far field focus may be done when $t_4/t_1 > 14$, whereas switching to near field focus may be done when $t_4/t_1 < 10$.

To have an idea of the required accuracy of $t_4/t_1$, the typical values of the parameters $p = 4$ mm, and $v = 0.2$ m/s may be used, and find $t_4 = 20$ ms. If discerning between the values 10, 12 and 14 for the ratio $t_4/t_1$ is required, then discerning between values 1.4, 1.7 and 2.0 for $t_1$ is needed. This implies an accuracy of 0.3 ms for the time measurement. A reasonable target accuracy may be 0.1 ms. This may be practically achieved using e.g. a low-power ASIC.

Returning now to Fig. 4A. In the above, it is assumed that the eyes behave symmetrically, in other words the subject looks straight ahead. If the subject instead looks skewed to the left or right, the processing units in one or both contact lenses 220 may determine that the position of the contact lens 220 with respect to the magnet trajectory is not centered, and that the viewing angle may be corresponding to that of an intended focus in the near field. In case the contact lenses 220 are configured to for example change a dioptric power, one or both contact lenses 220 may thus switch to near field focus erroneously.

As illustrated in Fig. 4A, optionally a third magnet 213 may be arranged on the eyelid 11. The third magnet 213 may be horizontally shifted with respect to the first 211 and second magnets 212. By the present arrangement, the third magnet 213 may follow a different magnet trajectory than the first 211 and the second magnets 212, during the blink movement of the eyelid 11. Thus, the third magnet 213 may pass over the edge 222 of the loop antenna 221 at a different location than the first 211 and second magnets 212.

The third magnet 213 may be arranged to be also vertically shifted with respect to the first 211 and second magnets 212. In this manner, it may be ensured that the third magnet 213 passes over the edge 222 at a different timepoint than the first 211 and second magnets 212, and thus three clearly distinguishable pulses may be induced in the loop antenna 221.

Optionally arranging the third magnet 213 on the eyelid 11 horizontally and vertically shifted from the two other magnets may allow the processing unit to determine whether the eye is shifted towards the nose or away from it. Thus, at least for an eye with a viewing angle turning away from the nose, the changing of dioptric power to near field focus may be avoided.

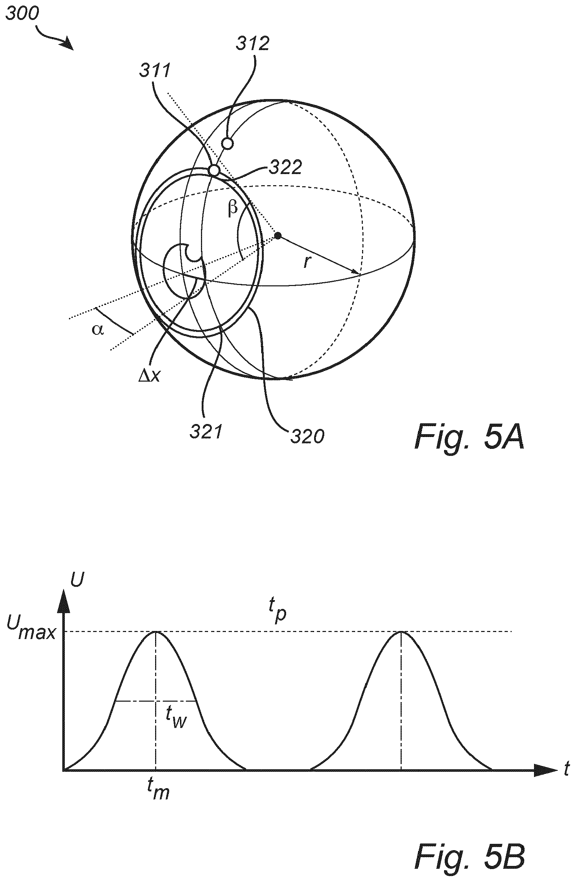

Fig. 5A schematically illustrates a contact lens system 300, comprising two magnets, i.e. a first magnet 311 and a second magnet 312, arranged on the outside of an eyelid 11 of a subject. The two magnets 311, 312 have a circular shape. The contact lens system 300 shares some of the features with the contact lens system 200 described in relation to Figs 4A-4E, the details of which are not repeated here.

As illustrated in Fig. 5A, the first magnet 311 and the second magnet 312 may be arranged on the eyelid 11, one above the other, such that the two magnets 311, 312 follow the same magnet trajectory during a blink movement of the eyelid 11. In Fig. 5A, $\Delta x$ denotes the horizontal displacement of the contact lens 320 with respect to the magnet trajectory. By the present arrangement, the first magnet 311 and the second magnet 312 pass over the edge 322 of the loop antenna 321 one after the other. In the present example, the two magnets 311, 312 are arranged on the eyelid 11 so that they typically pass only one edge 322, in this case the upper edge 322 of the loop antenna 321. For each of the two magnets 311, 312, passing the edge 322 of the loop antenna 321 causes a change of magnetic flux through the loop antenna 321, in response to which two pulses are induced.

Fig. 5B schematically illustrates the expected voltage waveform of pulses in response to the two circular magnets 311, 312 passing over the edge 322 of the loop antenna 321. The pulses induced by the circular magnets 311, 312 have a different shape as compared to the pulses induced by the rectangular magnets 211, 212. Each of the pulses show a certain rise time and a decay time, however, no clear plateau is reached therebetween.

As the horizontal displacement $\Delta x$ of the contact lens 320 increases, the pulses grow wider. Hence, the position of the contact lens 320 with respect to the magnet trajectory, and thus the intended focal distance, may be determined by the full width at half maximum (FWHM) of one pulse divided by the time difference between the voltage peak of the two pulses $t_p$.

Alternatively, the position of the contact lens 320 with respect to the magnet trajectory, and thus the intended focal distance, may be determined by measuring the time $t_p$ between the voltage peaks and the time from the start of the pulse to its maximum, $t_m$.

It serves to mention that the position of the contact lens 320 with respect to the magnet trajectory may also be determined using a single magnet, analyzing a single pulse during a blink movement. In a single pulse it may be seen that the skewness of the pulse increases with increasing $\Delta x$. More specifically, for larger $\Delta x$, the trailing edge of the pulse may be less steep than the leading edge of the pulse. For smaller $\Delta x$, this difference is smaller. The skewness of the pulse is a result of the circular magnet 311 entering the loop antenna 320. Thus, when a magnet 311 displaced from the center, enters the loop antenna 321, the magnetic flux increase in the beginning may be faster than the flux decrease at the end, leading to a skewed pulse.

In other words, by having the processing unit analyzing the shape of the pulse, the position of the contact lens 320 with respect to the magnet trajectory of the magnet 311 may be determined.

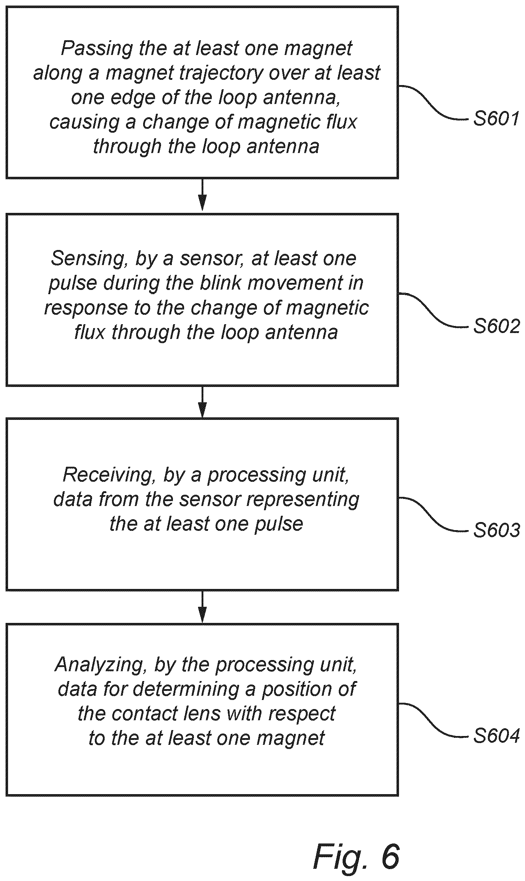

Fig. 6 illustrates a schematic block diagram shortly summarizing the method for determining a position of a contact lens with respect to at least one magnet of a contact lens system. The at least one magnet is arranged at an eyelid of a subject for moving with the eyelid. The contact lens comprising a loop antenna is arranged on an eyeball. It should be understood that the steps of the method, although listed in a specific order herein, may be performed in any order suitable.

The method may comprise passing S601, during a blink movement of the eyelid, the at least one magnet along a magnet trajectory over at least one edge of the loop antenna, causing a change of magnetic flux through the loop antenna.

The method may comprise sensing S602, by a sensor, at least one pulse during the blink movement in response to the change of magnetic flux through the loop antenna.

The method may comprise receiving S603, by a processing unit, data from the sensor representing the at least one pulse.

The method may comprise analyzing S604, by the processing unit, the data for determining a position of the contact lens with respect to the at least one magnet.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For example, in case the subject intentionally cross-eyes while wearing the contact lens, upon closing and reopening the eyelid, only one pulse will be detected each time, due to an overlap between the two consecutive pulses. This may enable the use of cross-eye movement as a command, allowing functionalities such as contact lens reset, forced switching to far field focus, or toggling between near field and far field focus.

**Claims**

1. A contact lens system (100, 200, 300) comprising:

   at least one magnet (110, 211, 212, 213, 311, 312) configured to be arranged at an eyelid (11) of a subject for moving with the eyelid (11);
   a contact lens (120, 220, 320) configured to be arranged on an eyeball (12), the contact lens (120, 220, 320) comprising a loop antenna (121, 221, 321), wherein the loop antenna (121, 221, 321) is configured to be arranged for intersecting with a magnet trajectory of the at least one magnet (110, 211, 212, 213, 311, 312), during a blink movement of the eyelid (11), wherein the at least one magnet (110, 211, 212, 213, 311, 312) passes over at least one edge (122, 222, 322) of the loop antenna (121, 221, 321), causing a change of magnetic flux through the loop antenna (121, 221, 321);
   a sensor (130) configured for sensing at least one pulse during the blink movement in response to the change of magnetic flux through the loop antenna (121, 221, 321); and
   a processing unit (140) configured to receive data from the sensor (130) representing the at least one pulse, and to analyze the data for determining a position of the contact lens (120, 220, 320) with respect to the magnet trajectory of the at least one magnet (110, 211, 212, 213, 311, 312).

2. The contact lens system (100, 200, 300) according to claim 1, wherein the processing unit (140) is further configured to send a control signal, based on the position of the contact lens (120, 220, 320) with respect to the magnet trajectory of at least one magnet (110, 211, 212, 213, 311, 312), to the contact lens (120, 220, 320).

3. The contact lens system (100, 200, 300) according to claim 2, wherein the contact lens (120, 220, 320) is configured to receive the control signal, and in response to the control signal change a dioptric power of the contact lens (120, 220, 320).

4. The contact lens system (100, 200, 300) according to any one of the preceding claims, wherein the loop antenna (121, 221, 321) extends along a perimeter of the contact lens (120, 220, 320).

5. The contact lens system (100, 200, 300) according to any one of the preceding claims, wherein the processing unit (140) is configured to determine the position of the contact lens (120, 220, 320) based on a shape of the at least one pulse.

6. The contact lens system (100, 200, 300) according to claim 5, wherein the processing unit (140) is configured to determine the position of the contact lens (120, 220, 320) based on a rise time of the at least one pulse.

7. The contact lens system (100, 200, 300) according to any one of the preceding claims, wherein

the sensor (130) is configured for sensing at least two pulses during the blink movement in response to the change of magnetic flux through the loop antenna (121, 221, 321); and wherein
the processing unit (140) is configured to receive data from the sensor (130) representing the at least two pulses, and to analyze the data for determining a position of the contact lens (120, 220, 320) with respect to the magnet trajectory of the at least one magnet (110, 211, 212, 213, 311, 312).

8. The contact lens system (100, 200, 300) according to claim 7, wherein the processing unit (140) is configured to determine the position of the contact lens (120, 220, 320) based on a time difference between the at least two pulses.

9. The contact lens system (200, 300) according to any one of claims 7 or 8, wherein the at least one magnet (211, 212, 213, 311, 312) comprises a first magnet (211, 311) and a second magnet (212, 312), and wherein the first magnet (211, 311) and the second magnet (212, 312) are configured to be arranged at the eyelid (11) such that the first magnet (211, 311) and the second magnet (212, 312), during the blink movement of the eyelid, (11), follow the same magnet trajectory and such that the first magnet (211, 311) and the second magnet (212, 312) pass over the at least one edge (222, 322) of the loop antenna (221, 321) one after the other, causing a change of magnetic flux through the loop antenna (221, 321), in response to which the at least two pulses are induced.

10. The contact lens system (200) according to claim 9, wherein the at least one magnet (211, 212, 213) further comprises a third magnet (213) configured to be arranged at the eyelid (11) such that the third magnet (213), during the blink movement of the eyelid (11), follows a different magnet trajectory than the first magnet (211) and the second magnet (212), and to pass over the at least one edge (222) of the loop antenna (221) at a different location from a location at which the first (211) and second magnets (212) pass over the at least one edge (222) of the loop antenna (221), causing a change of magnetic flux through the loop antenna (221).

11. The contact lens system (100) according to any one of claims 7 or 8, wherein the at least one magnet comprises a single magnet (110), and wherein the single magnet (110) is configured to be arranged at the eyelid (11) such that the single magnet (110), during the blink movement of the eyelid (11), follows the magnet trajectory to pass over a first edge (122') of the loop antenna (121) and a second edge (122") of the loop antenna (121), one after the other, causing a change of magnetic flux through the loop antenna (121), in response to which the at least two pulses are induced.

12. A contact lens (120, 220, 320) configured to be arranged on an eyeball, the contact lens (120, 220, 320) comprising:

a loop antenna (121, 221, 321), wherein the loop antenna (121, 221, 321) is configured to be arranged for a magnet trajectory of at least one magnet (110, 211, 212, 213, 311, 312) moving with an eyelid (11) during a blink movement, to pass over at least one edge (122, 222, 322) of the loop antenna (121, 221, 321), causing a change of magnetic flux through the loop antenna (121, 221, 321); and
a sensor (130) configured for sensing at least one pulse during the blink movement in response to the change of magnetic flux through the loop antenna (121, 221, 321).

13. The contact lens (120, 220, 320) according to claim 12, further comprising:
a processing unit (140) configured to receive data from the sensor (130) representing the at least one pulse, and to analyze the data for determining a position of the contact lens (120, 220, 320) with respect to the at least one magnet (110, 211, 212, 213, 311, 312).

14. A method for determining a position of a contact lens with respect to at least one magnet of a contact lens system, wherein the at least one magnet is arranged at an eyelid of a subject for moving with the eyelid, and wherein the contact lens is arranged on an eyeball, the contact lens comprising a loop antenna, the method comprising:

passing (S601), during a blink movement of the eyelid, the at least one magnet along a magnet trajectory over at least one edge of the loop antenna, causing a change of magnetic flux through the loop antenna;
sensing (S602), by a sensor, at least one pulse during the blink movement in response to the change of magnetic flux through the loop antenna;

receiving (S603), by a processing unit, data from the sensor representing the at least one pulse; and

analyzing (S604), by the processing unit, the data for determining a position of the contact lens with respect to the at least one magnet.

15. A processing unit (140) for determining a position of a contact lens (100, 200, 300) with respect to at least one magnet (110, 211, 212, 213, 311, 312) of a contact lens system (100, 200, 300), the processing unit (140) being configured to:

receive data, wherein the data represents at least one pulse induced in response to a change of magnetic flux through a loop antenna (121, 221, 321) of the contact lens (120, 220, 320) and sensed by a sensor (130), wherein the change of magnetic flux through the loop antenna (121, 221, 321) is caused by the at least one magnet (110, 211, 212, 213, 311, 312) being passed along a magnet trajectory over at least one edge (122, 222, 322) of the loop antenna (121, 221, 321), during a blink movement of the eyelid (11), while the at least one magnet (110, 211, 212, 213, 311, 312) is arranged at an eyelid (11) of a subject for moving with the eyelid (11), and while the contact lens (120, 220, 320) is arranged on an eyeball; and

analyze the data for determining a position of the contact lens (120, 220, 320) with respect to the at least one magnet (110, 211, 212, 213, 311, 312).

Fig. 1

Fig. 2A

Fig. 2B

Fig. 2C

Fig. 3

Fig. 4A

Fig. 4B

Fig. 4C

Fig. 4D

Fig. 4E

*Fig. 5A*

*Fig. 5B*

Passing the at least one magnet along a magnet trajectory over at least one edge of the loop antenna, causing a change of magnetic flux through the loop antenna — S601

Sensing, by a sensor, at least one pulse during the blink movement in response to the change of magnetic flux through the loop antenna — S602

Receiving, by a processing unit, data from the sensor representing the at least one pulse — S603

Analyzing, by the processing unit, data for determining a position of the contact lens with respect to the at least one magnet — S604

Fig. 6

Europäisches
Patentamt

European
Patent Office

Office européen
des brevets

# EUROPEAN SEARCH REPORT

**Application Number**

EP 24 18 3971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 285 803 B2 (LENSVECTOR INC [US]) 14 May 2019 (2019-05-14) | 1-15 | INV. G02C7/04 |
| Y | * figures 16A, 16B * * column 23, lines 14-15, 41-47, 55-58 * * column 9, lines 6-9 * ----- | 1-15 | G02C11/00 A61B3/113 |
| X | US 11 791 657 B2 (MOR RESEARCH APPLIC LTD [IL]; BLINK ENERGY LTD [IL]) 17 October 2023 (2023-10-17) | 1,4, 11-15 | |
| Y | * figures 16, 19, 22 * * column 21, lines 30-35 * ----- | 1-15 | |
| X | US 2018/043646 A1 (LAI HORNG JI [TW] ET AL) 15 February 2018 (2018-02-15) * figures 3A, 3B, 4A, 5 * * paragraphs [0029] - [0031] * ----- | 1,4, 11-15 | |
| X | US 2024/108456 A1 (COHEN NADAV [IL] ET AL) 4 April 2024 (2024-04-04) * figures 1B, 4A, 4B, 9A, 9B * * paragraph [0052] * ----- | 1,4, 11-15 | |
| | | | **TECHNICAL FIELDS SEARCHED (IPC)** G02C A61B |

The present search report has been drawn up for all claims

| Place of search | Date of completion of the search | Examiner |
|---|---|---|
| Munich | 25 November 2024 | de Lajarte, Gilles |

EPO FORM 1503 03.82 (P04C01)

# EP 4 671 853 A1

**ANNEX TO THE EUROPEAN SEARCH REPORT
ON EUROPEAN PATENT APPLICATION NO.**

EP 24 18 3971

This annex lists the patent family members relating to the patent documents cited in  the above-mentioned European search report.
The members are as contained in the European Patent Office EDP file on
The European Patent Office is in no way liable for these particulars which are merely given for the purpose of information.

25-11-2024

| Patent document cited in search report | | | Publication date | Patent family member(s) | | | Publication date |
|---|---|---|---|---|---|---|---|
| US 10285803 | B2 | | 14-05-2019 | US | 2016081793 | A1 | 24-03-2016 |
| | | | | WO | 201494432 | A1 | 11-12-2014 |
| US 11791657 | B2 | | 17-10-2023 | US | 2021226476 | A1 | 22-07-2021 |
| | | | | WO | 2019220307 | A1 | 21-11-2019 |
| US 2018043646 | A1 | | 15-02-2018 | CN | 107713998 | A | 23-02-2018 |
| | | | | GB | 2555904 | A | 16-05-2018 |
| | | | | JP | 2018041070 | A | 15-03-2018 |
| | | | | TW | 201809809 | A | 16-03-2018 |
| | | | | US | 2018043646 | A1 | 15-02-2018 |
| US 2024108456 | A1 | | 04-04-2024 | CN | 116615867 | A | 18-08-2023 |
| | | | | EP | 4247299 | A1 | 27-09-2023 |
| | | | | IL | 302680 | A | 01-07-2023 |
| | | | | JP | 2023552087 | A | 14-12-2023 |
| | | | | KR | 20230110268 | A | 21-07-2023 |
| | | | | US | 2024108456 | A1 | 04-04-2024 |
| | | | | WO | 2022107043 | A1 | 27-05-2022 |

EPO FORM P0459

For more details about this annex : see Official Journal of the European Patent Office, No. 12/82